# EUROPEAN PATENT SPECIFICATION

(11) **EP 2 032 046 B1**
(45) Date of publication and mention of the grant of the patent: **04.11.2015**
(21) Application number: 07872661.9
(22) Date of filing: 12.06.2007
(51) Int. Cl.: A61B 17/15, A61B 19/00, A61B 17/17

(54) **SYSTEMS AND DEVICES FOR TIBIAL RESECTION**
SYSTEME UND VORRICHTUNGEN ZUR SCHIENBEINGEWEBERESEKTION
SYSTÈMES ET DISPOSITIFS POUR UNE RÉSECTION TIBIALE

(30) Priority: 12.06.2006 US 812849 P
(43) Date of publication of application: 11.03.2009
(73) Proprietor: Smith & Nephew, Inc., Memphis, TN 38116 (US)
(72) Inventor: NADZADI, Mark, E., Memphis, Tennessee 38103 (US); SCIFERT, Chris F., Bartlett, Tennessee 38135 (US); KREUZER, Stefan, W., Huston, Texas 77024 (US)
(74) Representative: Smith & Nephew
(86) International application number: PCT/US2007/071042
(87) International publication number: WO 2008/091358

(56) References cited:
- EP-A- 0 337 901
- DE-A1- 10 309 493
- US-A- 5 628 749
- US-A- 5 810 829
- US-A1- 2003 100 906
- US-A1- 2003 212 403
- US-A1- 2005 234 465
- US-A1- 2005 273 113

## Description

### BACKGROUND

### FIELD OF THE INVENTION

The present invention relates to systems and devices for bone resection. More particularly, the present invention relates to tibial resection guides according to the appended claims.

### RELATED ART

Previous cutting instruments are all guided outside the bone, and once the instrument enters the bone it is no longer guided. Problems, such as tip deflection and skiving can affect bone cuts, implant alignment, and fixation. Previous instrumentation also will not fit under tight soft tissues, necessitating resection of those tissues or movement of those tissues.

Document US5628749 A discloses a tibial resection guide according to the preamble of claim 1.

### SUMMARY

A resection guide for a tibia comprises a first cutting guide and a second cutting guide. The first cutting guide is configured to overlay a portion of the tibia and to direct a cutting instrument in a plane. The first cutting guide has a length extending from a generally medial portion of the tibia to a generally lateral portion of the tibia. The first cutting guide has a depth extending in a posterior direction generally perpendicular to the length and a groove extending along the length and depth of the first cutting guide such that the groove extends along a generally transverse plane. The second cutting guide is oriented at an angle to the first cutting guide and configured to extend generally in a posterior direction from the first cutting guide. The second cutting guide limits the cutting instrument in the transverse plane from cutting bone.

Additionally, a resection guide may further comprise a third cutting guide configured to extend generally orthogonal to the transverse plane of the first cutting guide and oriented in the posterior direction of the second cutting guide.

Another embodiment may include a resection guide wherein the first and second cutting guides are configured to cut a medial portion of the tibia.

Additionally, a resection guide may further comprise a support structure. The support structure has a bone fixator configured to fixate the resection guide to the bone. The support structure may additionally include a cutting guide support configured to orient the varus/valgus angle of the first cutting guide.

Another embodiment may include a resection guide wherein the support structure has a port configured to receive a lateral resection guide.

One embodiment may include a resection guide wherein the lateral resection guide is fixed to the support structure with a connector.

Another embodiment may include a resection guide wherein the bone fixator is an extramedullary rod guide.

Additionally, the support structure may further comprise an offset configured to position the bone fixator away from the cutting guide support such that the bone fixator is outside the surgical field.

Additionally, the support structure may further comprise an offset configured to position the cutting guide support between the tibia and the patellar tendon and further position the bone fixator over the patellar tendon.

In one embodiment, the second cutting guide is a pin.

Additionally, the pin may include a cutout configured to retain the cutting instrument.

Another embodiment of the second cutting guide may include a sleeve having a cutout to retain the cutting instrument.

In another embodiment, the second cutting guide may extend into the tibia.

A method of resecting a portion of a tibia using the claimed resection guide includes the step of orienting a first cutting plane of a first cutting guide in a transverse plane in a medial/lateral direction. The first cutting plane sets the varus/valgus angle of the cutting plane. Another step installs a second cutting guide in the first cutting plane. The second cutting surface extends distally in the first cutting plane and limits the range of motion of a cutting instrument in the direction of the medial/lateral resection. Another step cuts the tibia from the outer surface of the bone along the first cutting plane in the first cutting guide to the second cutting guide.

Another method of using the guide of the invention further comprises the step of orienting a third cutting guide generally perpendicular to the first cutting plane. The third cutting guide extends in a sagittal plane. Another step includes cutting the tibia with the cutting instrument from a proximal portion along the sagittal plane of the third cutting guide distally until the cutting instrument contacts the second cutting guide.

In another method of using the guide of the invention, the installing step may further comprise the step of drilling a hole through the bone such that the hole is aligned along the first cutting plane.

Additionally, the installing step may further comprise the step of inserting a pin into the bone.

Additionally, the second cutting guide may be a sleeve on a pin. The installing step further comprises the step of removing the pin from the sleeve when the sleeve is placed in the bone.

In one embodiment the method further comprises the step of attaching a lateral cutting guide to the first cutting guide after the cutting step.

In another embodiment the method further comprises the step of fixing the cutting guide to the bone.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

The accompanying drawings, which are incorporated in and form a part of the specification, illustrate the embodiments of the present invention and together with the written description serve to explain the principles, characteristics, and features of the invention. In the drawings:
FIG. 1 is view of an embodiment of a tibial resection guide.
FIG. 2 is a view of a portion of the tibial resection guide of FIG. 1 placed on a tibia and receiving a cutting guide.
FIG. 3 is a view of the portion of the tibial resection guide of FIG. 2 with the cutting guide installed.
FIG. 4 is a view of an embodiment of a tibial resection guide for a medial resection.
FIG. 5 is a view of an embodiment of a lateral resection guide oriented relative to a medial resection.
FIG. 6 is another view of the embodiment of the lateral resection guide of FIG. 5.
FIG. 7 is a view of an embodiment of a portion of tibial resection guide for a medial resection.
FIG. 8 is another view of the embodiment of the tibial resection guide of FIG. 7.
FIG. 9 is a view of other portions of the tibial resection guide of FIG. 7.
FIG. 10 a view of an embodiment of a tibial resection guide.
FIG. 11 is an end view of an embodiment of a pin having a cutout portion.
FIG. 12 is an end view of another embodiment of a pin having a cutout portion.

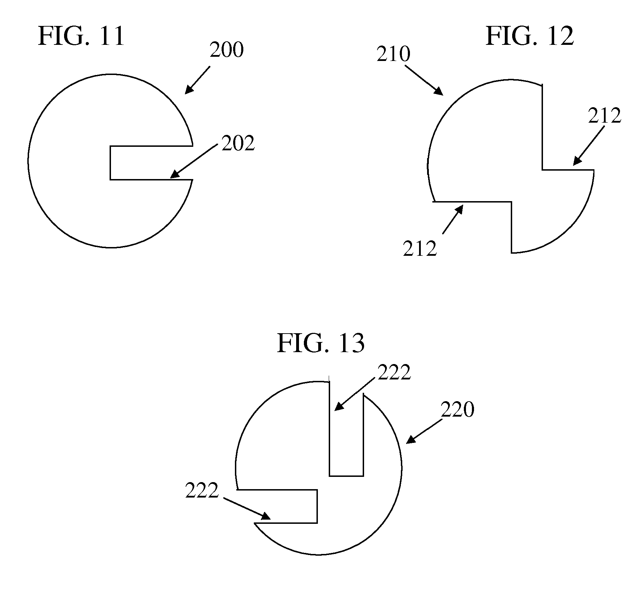

FIG. 12 is an end view of another embodiment of a pin having a cutout portion.

### DETAILED DESCRIPTION OF THE EMBODIMENTS

The following description of the preferred embodiment(s) is merely exemplary in nature and is in no way intended to limit the invention, its application, or uses.

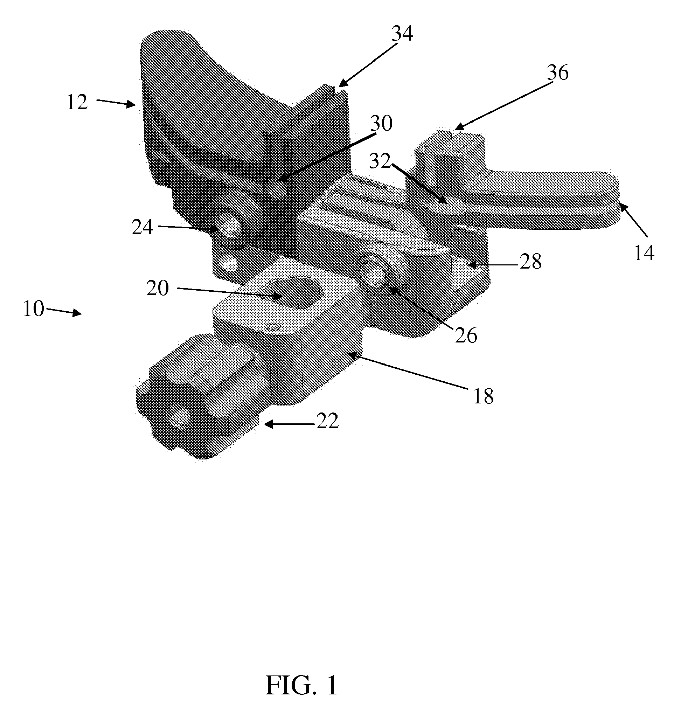

Turning to the drawing figures, FIG. 1 is view of an embodiment of a tibial resection guide 10. The guide 10 includes a medial cutting guide 12, a lateral cutting guide 14 and a bone fixator 18. The bone fixator 18, in this embodiment, is an extramedullary (EM) rod connector having an extramedullary rod guide 20 and a tightening knob 22. The medial and lateral cutting guides 12 and 14 include set screws 24 and 26 which lock the medial and lateral cutting guides 12 and 14 in place along a variable medial/lateral slots (slot 28 for the lateral side). Pin slots 30 and 32 in the cutting guides 12 and 14 are configured to receive cutting guides. Vertical cutting guides 34 and 36 are oriented above the pin slots 30 and 32.

The bone fixator 18 is configured to fix the cutting guide to the bone. The EM rod guide may be oriented to account for varus/valgus angle for the knee. Additionally, fixation pins may be used though openings in the resection guide 10 to fix the resection guide 10 to the bone. When the resection guide 10 is properly aligned and oriented on the EM rod, then the knob 22 may be tightened to fix the resection guide 10 in place.

The cutting guides 12 and 14 are oriented with respect to the bone fixator 18 to align the cutting surfaces for the medial and lateral portions of the tibia. The set screws 24 and 26 set the medial and lateral cutting guides 12 and 14 in place in the medial/ lateral direction. When the set screws 24 and 26 are loosened, then medial and lateral cutting guides may be variably positioned laterally and medially. Additionally, the set screws 24 and 26 may be removed to allow for the cutting guides 12 and 14 to be individually removed. The cutting guides 12 and 14 may be used independently, then, to minimize the size of the resection guide 10. A smaller resection guide 10 may help to minimize the incision size and minimize soft tissue resections or displacements.

The cutting guides 12 and 14 include horizontal cutting planes and vertical cutting planes. The horizontal and vertical cutting planes define the horizontal and vertical cutting surfaces for the tibia. When these guides 12 and 14 are used, the tibia will have medial and lateral resections with a shelf maintaining natural bone for the medial condyle between the resections where soft tissue may be maintained. For example, posterior and anterior cruciate ligaments attach to the tibia along the medial condyle of the tibia, and may be saved when the medial and lateral compartments are individually cut.

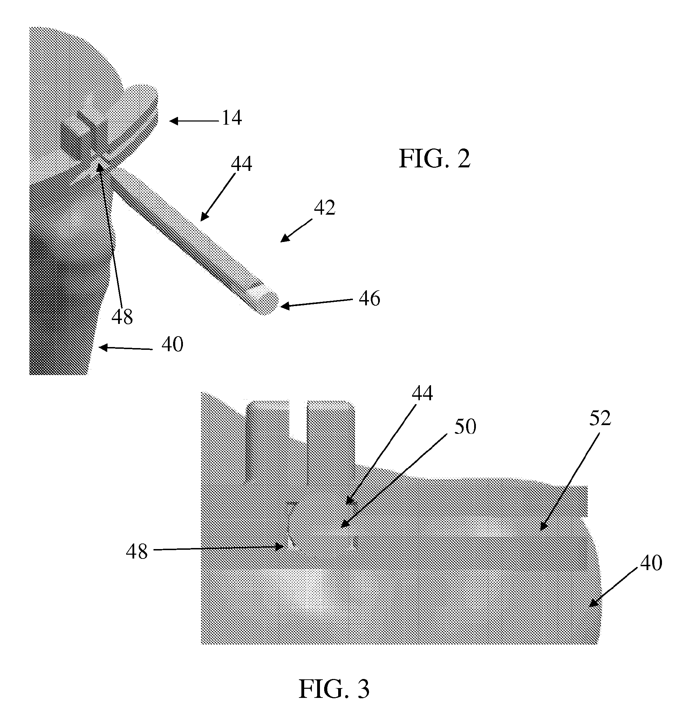

In operation, the guide 10 is placed on an EM rod and fixed to the rod. The angle of the rod fixes the varus/ valgus rotation of the resection guide 10. Generally, the medial compartment is resected first. The medial cutting guide 12 is positioned on the tibia. A pin (shown, for example, in FIG. 2) is inserted into the tibia. The horizontal cut in a transverse plane to the tibia is cut. The pin is set along the transverse plane. The vertical cutting guide 34 is positioned at an angle to the transverse plane and extends to the pin. Thus, the pin is in the transverse plane formed by the horizontal cutting guide and the sagittal plane of the vertical cutting guide. The cut in the transverse plane is cut to the pin, and the sagittal plane is cut down from above the tibia to the pin.

The pin cutting guide limits the cuts in both the transverse plane and sagittal plane. This minimizes the possibility of undercutting the resection. This also protects from overextending the cuts which may damage soft tissue. By creating a physical stop in the path of the cuts, the cuts may not extend past the stops. The pin may also provide a fillet at the corner to reduce stress risers in the bone. Additionally, as discussed below, the pin may limit tip deflection and better align or stabilize the cutting instruments during the cuts.

Turning now to FIG. 2, FIG. 2 is a view of a portion of the tibial resection guide 10 of FIG. 1 placed on a tibia 40 and receiving an anterior/posterior cutting guide 42. The lateral cutting guide 14 is positioned against a lateral portion of the tibia 40. The anterior/posterior cutting guide 42 may extend anterior/ posterior into the tibia and includes an outer sheath 44 and a pin 46. The anterior/posterior cutting guide 42 is inserted into a receiving slot 48 in the transverse guide of the lateral cutting guide 14. The anterior/posterior cutting guide 42 may be driven into the bone. Alternatively, a hole may be drilled into the bone and the anterior/posterior cutting guide 42 may be inserted into the receiving slot 48. Once the anterior/posterior cutting guide 42 is positioned within the receiving slot 48, the pin 46 may be removed from the sheath 44 leaving a cutting slot, as shown in FIG. 3.

Turning now to FIG. 3, FIG. 3 is a view of the portion of the tibial resection guide of FIG. 2 with the cutting guide 42 installed. The pin of the anterior posterior cutting guide 42 has been removed leaving the sheath 44 in the receiving slot 48. An A/P sheath slot 50 extends from the anterior portion of the tibia 40 to the posterior portion of the tibia 40. The sheath slot 50 allows for a cutting instrument to be inserted along the sheath slot 50 to start the cut and control the tip of the cutting instrument. The cut continues within a horizontal slot 52 to the lateral side of the tibia. The sheath slot 50 also stabilizes the transverse cut and properly aligns the transverse cut. As the transverse cut is made, the cut may not extend more medially than the sheath slot 50.

A vertical slot 54 extends from a proximal portion of the tibia 40 to the sheath 44. In this embodiment, the sheath is oriented as a stop and does not create a starting point inferiorly for the vertical cut. However, the receiving slot 48 and the sheath slot 50 may be oriented so that the vertical slot 54 may be aligned with the sheath slot 50.

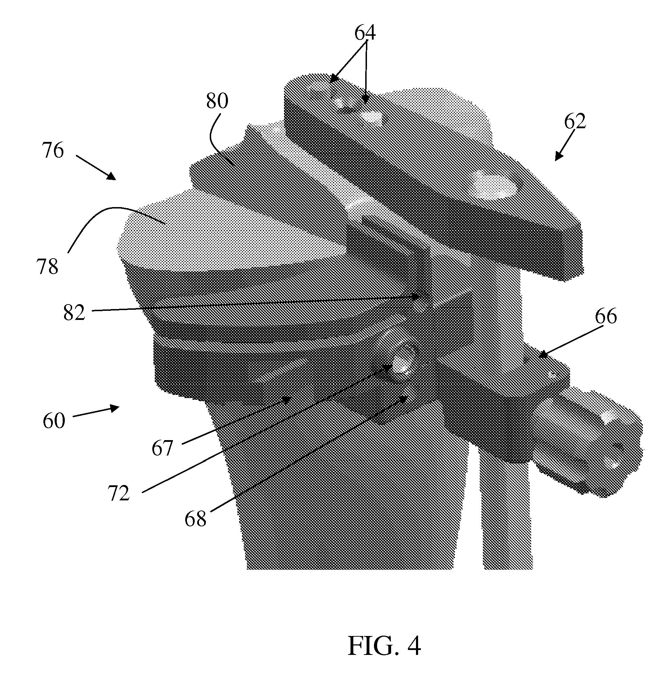

Turning now to FIG. 4, FIG. 4 is a view of an embodiment of a tibial resection guide 60 for a medial resection. The tibial resection guide 60 is connected to the bone with a spike rod 62. The spike rod 62 attaches to an EM guide 66 of the tibial resection guide 60. Pins 64 attach the medial cutting guide to the bone. Additional bone fixation may be achieved with a pin through a pinhole 68. A variable M/L slot 67 allows for the medial guide 60 to be moved medially and laterally with respect to the tibia, and is thus similar to the slot 28 of FIG. 1. A set screw 72 fixes the M/L position of the cutting guide 60.

The pins 64 may be of different heights so that a first pin may be hammered into the tibia first, and then the spike rod (and thus the EM rod guide 66 and cutting guide 60) may be rotated before the second, shorter pin 64 is knocked into the bone. Once the second shorter pin is placed, the guide 60 is fixed to the bone.

A medial cutout 76 shows the transverse cut 78 and the vertical cut 80 of the tibia. An A/P pin slot 82 is positioned so that an A/P pin would be placed at the intersection of the transverse cut 78 and the vertical cut 80 so that when the transverse and vertical cuts are made, the A/P pin would limit the lateral edge of the transverse cut and the inferior edge of the vertical cut.

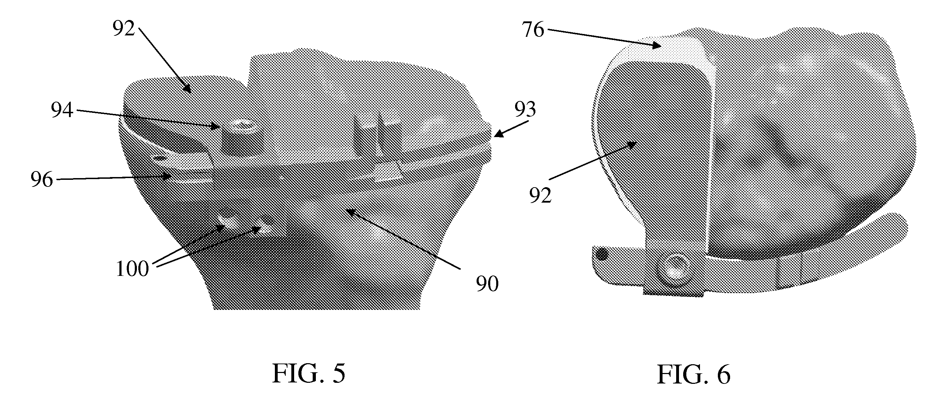

Turning now to FIG. 5, FIG. 5 is a view of an embodiment of a lateral resection guide 90 oriented relative to a medial resection. For example, the lateral resection guide 90 may be used with the medial resection guide of FIG. 4. The lateral guide 90 includes a medial pad 92 which orients a lateral cutting guide 93 to the tibia. A set screw 94 sets the medial lateral position of the lateral resection guide 90 in a slide 96. Fixation is achieved by extending the knee and having the medial condyle of the femur pressing down on the medial pad 92. Additionally, pinholes 100 may be used to fixate the lateral resection guide 90 to the bone. Extending the femur also relaxes the patella, moving the patella out of the way for lateral cuts. The horizontal and vertical cutting guides for the lateral resections are operated similar to the cuts discussed previously.

The cuts may be formed either using a sagittal saw or a reciprocating saw. When using a reciprocating saw, then the preferred cut starts at the A/P guide and progresses toward the posterior of the tibia within the slot of the A/P guide. After completing a cut from the anterior to the posterior of the tibia, then the blade is pushed out within the transverse slot of the lateral guide 94.

Turning now to FIG. 6, FIG. 6 is another view of the embodiment of the lateral resection guide of FIG. 5. The medial pad 92 sits on the medial tibial cutout 76. Any variation in the medial cut 76 will be transferred to the lateral side through the medial pad 92. Thus, misalignment between the medial and lateral cuts is minimized through the sequential cutting of first the medial than the lateral compartments.

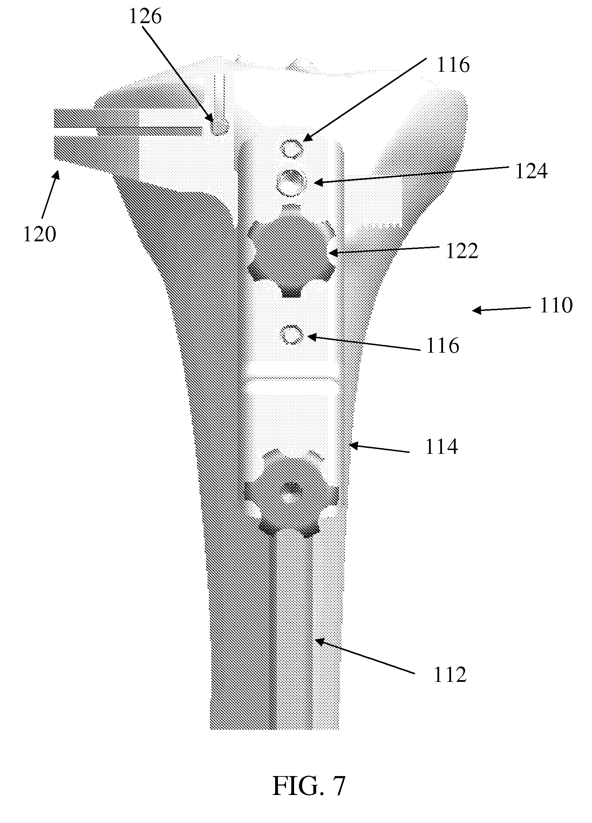

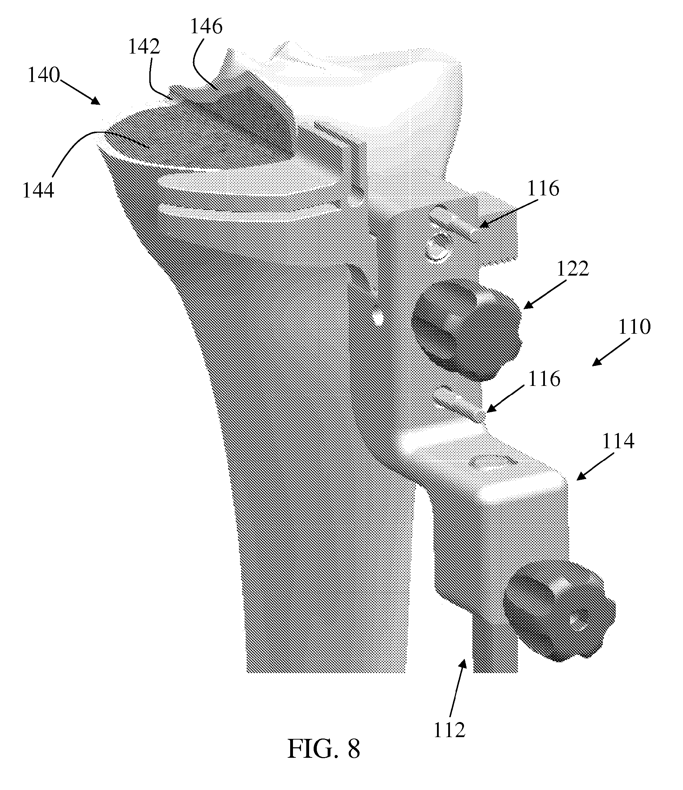

Turning now to FIGs. 7 and 8, FIG. 7 is a view of an embodiment of a portion of tibial resection guide 110 for a medial resection. An extra medullary rod 112 fixates the resection guide 110 with a step down block 114. Pins 116 further fixate the guide 110 to the bone. A medial cutting guide 120 may be moved medially/laterally with a knob 122 and set in place by a set screw 124. An A/P cutting guide 126 through the medial cutting guide 120 limits the cuts of a medial cutout. FIG. 8 is another view of the embodiment of the tibial resection guide 110 of FIG. 7. A medial cutout 140 shows a filleted cut surface 142 at the intersection of a transverse cut 144 and a vertical cut 146

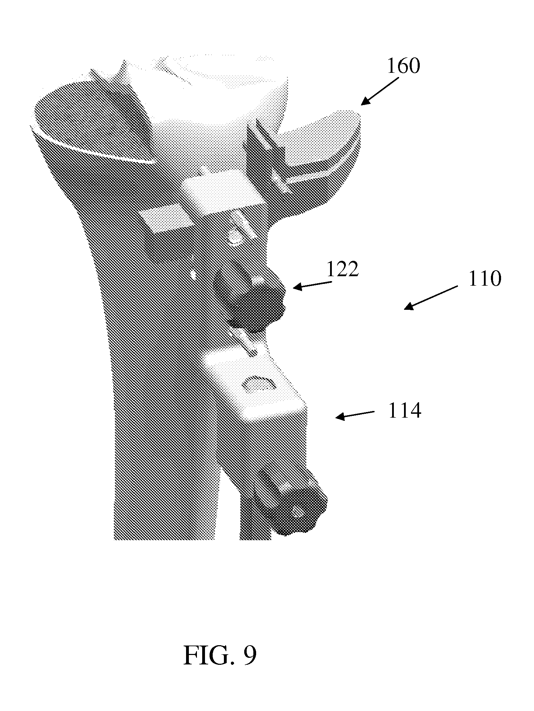

Turning now to FIG. 9, FIG. 9 is a view of other portions of the tibial resection guide 110 of FIG. 7. A lateral cutting guide 160 is received in the same slot as the medial guide of FIGs. 7 and 8. The knob 122 may orient the lateral cutting guide 160 in the medial/ lateral direction. Because the resection guide 110 is fixed in the same orientation as when the medial cuts were made, the relative orientation of the medial cuts to the lateral cuts are consistent.

The step down block 114 is an offset that moves part of the bone fixation and the part that orients the guide 110 away from the cutting planes and cutting surfaces. This allows for more access and added views of the cutting planes.

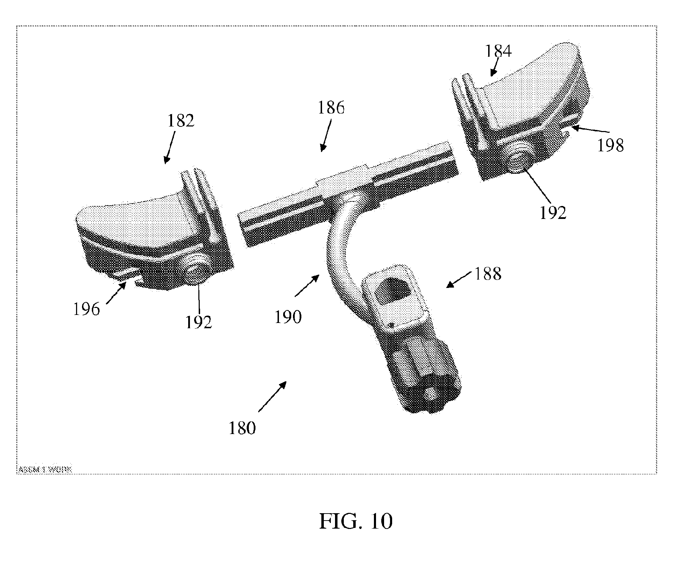

Turning now to FIG. 10, FIG. 10 a view of an embodiment of a tibial resection guide 180. A medial cutting guide 182 and a lateral cutting guide 184 are received on a cutting guide support. In this embodiment, the cutting guide support is a guide post 186. A bone fixator 188 is connected to the post 186 by an offset 190. Set screws 192 fix the cutting guides 182 and 184 to the post 186. The post 186 allows for medial/ lateral movement of the cutting guides 182 and 184 along tracks 196 and 198 respectively.

The tibial resection guide 180 is generally smaller in size than conventional resection guides. The offset 190 is shaped to allow for the post 186 to be inserted within an incision while the bone fixator 188 remains outside the incision. The curve in the offset 190 positions the post 186 between the tibia and the patellar tendon and position the bone fixator 188 over the patellar tendon. In order to minimize the size of the resection guide 180, the cutting guides 182 and 184 may be used individually.

Turning now to FIGs. 11-13, FIG. 11 is an end view of an embodiment of a pin 200 having a cutout portion 202. FIG. 12 is an end view of another embodiment of a pin 210 having a cutout portions 212. FIG. 12 is an end view of another embodiment of a pin 220 having a cutout portions 222. Each of these pins 200, 210, and 220 may be used as pins with the embodiments of resection guides previously discussed. The cutout portion 202 is a portion that is limited to a single slot in the A/P direction of the pin 200. The cutout portions 212 and 222 are configured to capture a cutting instrument in orthogonal planes in the A/P direction. While cutout portions 212 extend tangential to the cross section of the pin 210, the cutout portions 222 extend along secants of the cross section of the pin 220. While the cutouts 212 and 222 are positioned orthogonally, the cutouts 212 and 222 may be positioned at any angle relative to one another. Additionally, sheaths may be used with the pins to fill the cutouts initially if the pins are driven into the bone instead of placed within pre-drilled holes.

As various modifications could be made to the exemplary embodiments, as described above with reference to the corresponding illustrations, without departing from the scope of the invention, it is intended that all matter contained in the foregoing description and shown in the accompanying drawings shall be interpreted as illustrative rather than limiting. Thus, the breadth and scope of the present invention should not be limited by any of the above-described exemplary embodiments, but should be defined only in accordance with the following claims appended hereto and their equivalents.

## Claims

1. A resection guide (10) for a tibia, comprising:
an anterior/posterior cutting guide (42); and
a transverse cutting guide (12,14) configured to overlay a portion of the tibia and having a length extending from a generally medial portion of the tibia to a generally lateral portion of the tibia and a depth extending in a posterior direction generally perpendicular to the length, the first cutting guide comprising a groove (52) extending along the length and depth of the first cutting guide and along a generally transverse plane;
**characterised in that** the transverse cutting guide (12,14) further comprises at least one receiving slot (48) for receiving the anterior/posterior cutting guide (42), wherein the anterior/posterior cutting guide (42), when received in the at least one receiving slot (48), is oriented at an angle to the transverse cutting guide (12,14), extends generally in a posterior direction from the transverse cutting guide (12,14) and is configured to limit a cutting instrument from cutting bone in the transverse plane.

2. The resection guide of claim 1, further comprising a vertical cutting guide (34, 36) configured to extend generally orthogonal to the transverse cutting guide (12,14) and oriented in the anterior/posterior direction.

3. The resection guide of claim 2, wherein the at least one receiving slot (48) is located at an intersection of the transverse cutting guide (12,14) and the vertical cutting guide (34,36).

4. The resection guide of any preceding claim, wherein the transverse and anterior/posterior cutting guides are configured to cut a medial portion of the tibia.

5. The resection guide of any preceding claim, further comprising a support structure, the support structure having a bone fixator (188) configured to fixate the resection guide (10) to the bone, and a cutting guide support (186) configured to orient the varus/valgus angle of the transverse cutting guide.

6. The resection guide of claim 5, wherein the support structure comprises a port configured to receive a lateral resection guide (90).

7. The resection guide of claim 6, wherein the lateral resection guide (90) is fixed to the support structure with a connector.

8. The resection guide of any of claims 5-7, wherein the bone fixator is an extramedullary rod guide (112).

9. The resection guide of any of claims 5-8, wherein the support structure further comprises an offset (114) configured to position the bone fixator away from the cutting guide support such that the bone fixator is outside the surgical field.

10. The resection guide of any of claims 5-8, wherein the support structure further comprises an offset (190) configured to position the cutting guide support between the tibia and the patellar tendon and position the bone fixator over the patellar tendon.

11. The resection guide of any preceding claim, wherein the anterior/posterior cutting guide (42) comprises a pin (200, 210, 220).

12. The resection guide of claim 11, wherein the pin comprises a cutout (202, 212, 222) configured to retain a cutting instrument.

13. The resection guide of any preceding claim wherein the anterior/posterior cutting guide (42) comprises a sleeve (44) having a cutout (50) to retain a cutting instrument.

14. The resection guide of claim 12 or 13, wherein the cutout (202, 212, 222 or 50) comprises a slot.

15. The resection guide of any preceding claim, wherein the anterior/posterior cutting guide (42) is adapted to extend into the tibia.

## Patentansprüche

1. Eine Resektionsführung (10) für eine Tibia, die Folgendes beinhaltet:
eine anteriore/posteriore Schneidführung (42); und
eine transversale Schneidführung (12, 14), die so konfiguriert ist, dass sie einen Abschnitt der Tibia überlagert, und die eine Länge aufweist, die sich von einem im Allgemeinen medialen Abschnitt der Tibia bis zu einem im Allgemeinen lateralen Abschnitt der Tibia erstreckt, und eine Tiefe aufweist, die sich in einer posterioren, im Allgemeinen perpendikulär zu der Länge stehenden Richtung erstreckt, wobei die erste Schneidführung eine Kerbe (52) beinhaltet, die sich entlang der Länge und Tiefe der ersten Schneidführung und entlang einer im Allgemeinen transversalen Ebene erstreckt;
**dadurch gekennzeichnet, dass** die transversale Schneidführung (12, 14) ferner mindestens einen Aufnahmeschlitz (48) zum Aufnehmen der anterioren/posterioren Schneidführung (42) beinhaltet, wobei die anteriore/posteriore Schneidführung (42), wenn sie in dem mindestens einen Aufnahmeschlitz (48) aufgenommen wird, in einem Winkel zu der transversalen Schneidführung (12, 14) ausgerichtet ist, sich im Allgemeinen in einer posterioren Richtung von der transversalen Schneidführung (12, 14) erstreckt und so konfiguriert ist, dass sie das Schneiden von Knochen in der transversalen Ebene durch ein Schneidinstrument einschränkt.

2. Resektionsführung gemäß Anspruch 1, die ferner eine senkrechte Schneidführung (34, 36) beinhaltet, die so konfiguriert ist, dass sie sich im Allgemeinen rechtwinklig zu der transversalen Schneidführung (12, 14) erstreckt, und die in der anterioren/posterioren Richtung ausgerichtet ist.

3. Resektionsführung gemäß Anspruch 2, wobei der mindestens eine Aufnahmeschlitz (48) an einer Kreuzung der transversalen Schneidführung (12, 14) und der senkrechten Schneidführung (34, 36) angeordnet ist.

4. Resektionsführung gemäß einem der vorhergehenden Ansprüche, wobei die transversale und die anteriore/posteriore Schneidführung so konfiguriert sind, dass sie einen medialen Abschnitt der Tibia schneiden.

5. Resektionsführung gemäß einem der vorhergehenden Ansprüche, die ferner eine Stützstruktur beinhaltet, wobei die Stützstruktur eine Knochenfixiervorrichtung (188), die so konfiguriert ist, dass sie die Resektionsführung (10) an dem Knochen fixiert, und eine Schneidführungsstütze (186), die zum Ausrichten des Varus/Valgus-Winkels der transversalen Schneidführung konfiguriert ist, aufweist.

6. Resektionsführung gemäß Anspruch 5, wobei die Stützstruktur eine Öffnung beinhaltet, die zum Aufnehmen einer lateralen Resektionsführung (90) konfiguriert ist.

7. Resektionsführung gemäß Anspruch 6, wobei die laterale Resektionsführung (90) mit einem Verbindungsstück an der Stützstruktur fixiert ist.

8. Resektionsführung gemäß einem der Ansprüche 5-7, wobei die Knochenfixiervorrichtung eine extramedulläre Stabführung ist (112).

9. Resektionsführung gemäß einem der Ansprüche 5-8, wobei die Stützstruktur ferner ein Abstandsstück (114) beinhaltet, das zum Positionieren der Knochenfixiervorrichtung im Abstand von der Schneidführungsstütze konfiguriert ist, so dass die Knochenfixiervorrichtung sich außerhalb des chirurgischen Felds befindet.

10. Resektionsführung gemäß einem der Ansprüche 5-8, wobei die Stützstruktur ferner ein Abstandsstück (190) beinhaltet, das zum Positionieren der Schneidführungsstütze zwischen der Tibia und der Patellasehne und zum Positionieren der Knochenfixiervorrichtung über der Patellasehne konfiguriert ist.

11. Resektionsführung gemäß einem der vorhergehenden Ansprüche, wobei die anteriore/posteriore Schneidführung (42) einen Stift (200, 210, 220) beinhaltet.

12. Resektionsführung gemäß Anspruch 11, wobei der Stift einen Ausschnitt (202, 212, 222) beinhaltet, der zum Festhalten eines Schneidinstruments konfiguriert ist.

13. Resektionsführung gemäß einem der vorhergehenden Ansprüche, wobei die anteriore/posteriore Schneidführung (42) eine Hülse (44) mit einem Ausschnitt (50) zum Festhalten eines Schneidinstruments beinhaltet.

14. Resektionsführung gemäß Anspruch 12 oder 13, wobei der Ausschnitt (202, 212, 222 oder 50) einen Schlitz beinhaltet.

15. Resektionsführung gemäß einem der vorhergehenden Ansprüche, wobei die anteriore/posteriore Schneidführung (42) dazu angepasst ist, sich in die Tibia zu erstrecken.

## Revendications

1. Un guide de résection (10) pour un tibia, comprenant :
un guide de coupe antérieur/postérieur (42) ; et
un guide de coupe transversal (12, 14) configuré pour recouvrir une portion du tibia et ayant une longueur s'étendant d'une portion généralement médiale du tibia à une portion généralement latérale du tibia et une profondeur s'étendant dans une direction postérieure généralement perpendiculaire à la longueur, le premier guide de coupe comprenant une rainure (52) s'étendant sur la longueur et la profondeur du premier guide de coupe et sur un plan généralement transversal ;
**caractérisé en ce que** le guide de coupe transversal (12, 14) comprend en outre au moins une fente de réception (48) pour recevoir le guide de coupe antérieur/postérieur (42), le guide de coupe antérieur/postérieur (42), lorsqu'il est reçu dans l'au moins une fente de réception (48), étant orienté à un angle par rapport au guide de coupe transversal (12, 14), s'étendant dans une direction postérieure depuis le guide de coupe transversal (12, 14) et étant configuré pour limiter un instrument de coupe de couper un os dans le plan transversal.

2. Le guide de résection de la revendication 1, comprenant en outre un guide de coupe vertical (34, 36) configuré pour s'étendre de façon généralement orthogonale par rapport au guide de coupe transversal (12, 14) et orienté dans la direction antérieure/postérieure.

3. Le guide de résection de la revendication 2, dans lequel l'au moins une fente de réception (48) est située au niveau d'une intersection du guide de coupe transversal (12, 14) et du guide de coupe vertical (34, 36).

4. Le guide de résection de n'importe quelle revendication précédente, dans lequel les guides de coupe transversal et antérieur/postérieur sont configurés pour couper une portion médiale du tibia.

5. Le guide de résection de n'importe quelle revendication précédente, comprenant en outre une structure de support, la structure de support ayant un fixateur d'os (188) configuré pour fixer le guide de résection (10) sur l'os, et un support de guide de coupe (186) configuré pour orienter l'angle varus/valgus du guide de coupe transversal.

6. Le guide de résection de la revendication 5, dans lequel la structure de support comprend un orifice configuré pour recevoir un guide de résection latéral (90).

7. Le guide de résection de la revendication 6, dans lequel le guide de résection latéral (90) est fixé sur la structure de support grâce à un élément de raccordement.

8. Le guide de résection de n'importe lesquelles des revendications 5 à 7, dans lequel le fixateur d'os est un guide-broche extramédulaire (112).

9. Le guide de résection de n'importe lesquelles des revendications 5 à 8, dans lequel la structure de support comprend en outre un décalage (114) configuré pour positionner le fixateur d'os à l'écart du support de guide de coupe de telle sorte que le fixateur d'os se trouve à l'extérieur du champ chirurgical.

10. Le guide de résection de n'importe lesquelles des revendications 5 à 8, dans lequel la structure de support comprend en outre un décalage (190) configuré pour positionner le support de guide de coupe entre le tibia et le tendon rotulien et positionner le fixateur d'os par-dessus le tendon rotulien.

11. Le guide de résection de n'importe quelle revendication précédente, dans lequel le guide de coupe antérieur/postérieur (42) comprend une tige (200, 210, 220).

12. Le guide de résection de la revendication 11, dans lequel la tige comprend une découpe (202, 212, 222) configurée pour retenir un instrument de coupe.

13. Le guide de résection de n'importe quelle revendication précédente dans lequel le guide de coupe antérieur/postérieur (42) comprend un manchon (44) ayant une découpe (50) pour retenir un instrument de coupe.

14. Le guide de résection de la revendication 12 ou de la revendication 13, dans lequel la découpe (202, 212, 222 ou 50) comprend une fente.

15. Le guide de résection de n'importe quelle revendication précédente, dans lequel le guide de coupe antérieur/postérieur (42) est conçu pour s'étendre jusque dans le tibia.
